# EUROPEAN PATENT SPECIFICATION

(11) **EP 3 772 537 B1**
(45) Date of publication and mention of the grant of the patent: **15.10.2025**
(21) Application number: 20189880.6
(22) Date of filing: 06.08.2020
(51) Int. Cl.: C12N 5/00, G01N 33/50, C12N 5/077, A61K 35/16

(54) **USE OF A PLATELET DERIVATIVE FOR STIMULATING CELL PROLIFERATION**
VERWENDUNG EINES BLUTPLÄTTCHENDERIVATS ZUR STIMULIERUNG DER ZELLPROLIFERATION
UTILISATION D'UN DÉRIVÉ DE PLAQUETTES POUR STIMULER LA PROLIFÉRATION CELLULAIRE

(30) Priority: 08.08.2019 IT 201900014430
(43) Date of publication of application: 10.02.2021
(73) Proprietor: Medica S.p.A., 41036 Medolla (IT)
(72) Inventor: MAZZUCCO, Laura, 15033 Casale Monferrato (AL) (IT); BOCCHI, Letizia, 41036 Medolla (MO) (IT); DI SALVO, Maria Teresa, 41036 Medolla (MO) (IT)
(74) Representative: Studio Torta S.p.A.

(56) References cited:
- WO-A1-2014/076200
- WO-A1-2014/126931
- WO-A1-2019/123259
- WO-A1-93/24157
- WO-A2-2010/122548
- US-A- 5 580 465
- US-A1- 2002 179 537
- L. MAZZUCCO ET AL: "Not every PRP-gel is born equal Evaluation of growth factor availability for tissues through four PRP-gel preparations: Fibrinet , RegenPRP-Kit , Plateltex and one manual procedure", VOX SANGUINIS., vol. 97, no. 2, 1 August 2009 (2009-08-01), CH, pages 110 - 118, XP055688507, ISSN: 0042-9007, DOI: 10.1111/j.1423-0410.2009.01188.x
- ASLI KOCAOEMER ET AL: "Human AB Serum and Thrombin-Activated Platelet-Rich Plasma Are Suitable Alternatives to Fetal Calf Serum for the Expansion of Mesenchymal Stem Cells from Adipose Tissue", STEM CELLS, vol. 25, no. 5, 1 May 2007 (2007-05-01), pages 1270 - 1278, XP055060990, ISSN: 1066-5099, DOI: 10.1634/stemcells.2006-0627
- ANJA GOEDECKE ET AL: "Differential effect of platelet-rich plasma and fetal calf serum on bone marrow-derived human mesenchymal stromal cells expanded in vitro", JOURNAL OF TISSUE ENGINEERING AND REGENERATIVE MEDICINE, vol. 5, no. 8, 1 August 2011 (2011-08-01), US, pages 648 - 654, XP055688532, ISSN: 1932-6254, DOI: 10.1002/term.359

## Description

### TECHNICAL FIELD

The present invention concerns a method for preparing a plasma enriched with platelets and plasma proteins to stimulate cell proliferation and as a supplement for a cell culture medium.

### BACKGROUND ART

The repair and regeneration of a tissue entail a set of complex phases and the role of growth factors in inducing and accelerating these mechanisms is not fully defined.

By regeneration we mean reconstitution of the form and function of a damaged tissue; by repair, on the other hand, the return to continuity of the tissue, without necessarily reintegrating its original architecture and, above all, without integral restoration of its function.

The use of platelet concentrates is known, including platelet-rich plasma (or PRP), for tissue repair and regeneration.

When describing the action of the platelet concentrates, the regenerative effect is commonly attributed to the platelet growth factors.

Proteomics studies have identified over 2000 biologically active proteins derived from platelets and involved in the mechanisms of: chemotaxis, cell proliferation, angiogenesis, deposition of extracellular matrix (ECM) and tissue repair. These are directly induced by the platelet factors (growth factors, angiopoietin, SDF-1, MMP-1,-2 and -9) and indirectly by chemokines and cytokines produced by bystander cells (fibroblasts, macrophages, endothelial cells and lymphocytes), the action of which is triggered by a complex network of gene activations.

One of the first works comparing different methods for preparing platelet-rich plasma was "Not every PRP-gel is born equal. Evaluation of growth factor availability for tissues through four PRP-gel preparations: Fibrinet^{®}, RegenPRP-KIT^{®}, Plateltex^{®} and one manual procedure" (Mazzucco et al., Vox Sang. 2009 Aug; 97(2):110-8).

In this publication it was hypothesized that PRP prepared with different methods and therefore with distinct characteristics in terms of platelet concentration and platelet growth factor release kinetics could be applied in different clinical settings.

These evaluations had led to the conclusion that methods and systems of preparation, although substantially similar, can induce secondary effects on the platelets and therefore modulate and modify the final release of the growth factors. The consequent variability in the release of growth factors and bioactive substances can variously affect the response mechanisms of the tissues treated with derivatives of the PRP, conditioning variable tissue regeneration responses. However, there is no clinical evidence of this important point.

The bioavailability of the molecules present in the different products obtained by the methods examined has indicated and led to the conclusion that it would be possible to optimize the suitability for use of the different PRP by identifying platelet concentrates specific for each clinical condition. However, this ambitious therapeutic objective remains unfulfilled.

From 1998 to today, from the simple platelet-rich plasma defined by Marx, multiple types of preparations of these blood components have been developed; they are differentiated by the product characteristics but can be grouped into: pure platelet concentrates, platelet concentrates enriched with fibrin, with or without leukocytes.

The pure platelet concentrate is a platelet-rich plasma. The concentration of the platelets is variable but must be higher than the normal value of the peripheral blood (3 to 6 times). It can have a very low leukocyte content, below 0.5 per µL. It can be obtained by centrifugation of the anticoagulated blood (ACD or Sodium Citrate) with specific protocols or dedicated devices.

The platelet concentrate enriched with fibrin is a platelet concentrate that has a high fibrin density and is in gel form. It is obtained by direct centrifugation of the blood, generally without anticoagulant. The concentration of platelets is difficult to evaluate since they are trapped in the fibrin and in the coagulate.

Both the pure platelet concentrate and the platelet concentrate enriched with fibrin can be enriched with leukocytes. The rheology of the blood induces a stratification of the cell elements by dimension and specific weight. The leukocytes are positioned in the buffy coat which is located between the plasma, in which the platelets remain in suspension, and the red blood cells. By recovering this fraction, it is possible to obtain a non-selective contamination of the platelet concentrate (CP) since the leukocyte fraction is global and therefore contains lymphocytes, monocytes and neutrophils.

Nevertheless, the need is felt in the art for new platelet concentrates which have improved properties in terms of inducing cell proliferation.

An object of the present invention is therefore to provide new platelet concentrates able to increase the cell proliferation levels compared to the known platelet concentrates.

### DISCLOSURE OF INVENTION

The object of the present invention is achieved by a method for the preparation of plasma enriched with platelets and plasma proteins according to claim 1.

In particular, according to a first aspect of the invention, a method is provided for preparing a plasma enriched with platelets and plasma proteins for use in stimulating cell proliferation in which said plasma enriched with platelets and plasma proteins has a platelet content of at least 600000 platelets/µL, preferably 800000-1600000 platelets/µL and a plasma protein content of at least 10 g/dL, preferably 12-18 g/dL.

In accordance with a second aspect of the disclosure, the plasma thus obtained can also be used as a supplement for a cell culture medium.

The plasma enriched with platelets and plasma proteins of the invention is obtained via the steps of:
- obtaining a platelet-rich plasma by means of centrifugation or extraction from anticoagulated whole blood or from platelet concentrate from apheresis;
- filtering said platelet-rich plasma on hollow fibres of polysulfone or derivatives thereof having an outer surface provided with pores having mean diameter ranging from 5 µm to 15 µm and an inner surface provided with pores having mean diameter ranging from 5 nm to 80 nm to obtain a plasma enriched with platelets and plasma proteins.

By the term "plasma enriched with platelets and plasma proteins" we mean a plasma in which the platelet levels are at least three times higher than the basal levels of the circulating blood and the plasma protein levels are at least twice as high as the basal levels of the circulating blood.

Advantageously, filtering of the plasma on polysulfone allows concentration of the platelets and plasma proteins due to elimination of part of the aqueous component of the plasma. Said dead-end filtering process under controlled pressure, together with the haemocompatibility of the filtering material, ensure that the platelets are not damaged and minimize the interaction between the blood components and the biomaterial.

The plasma component provides important proteins such as fibrinogen, fibronectin, vitronectin, Von Willebrand factor, thrombospondin-1 and 2, plasminogen activator inhibitor 1 (PAI-1), platelet factor 4 (PF4), proteins with adhesivechemotactic action for monocytes and neutrophils and with immunoregulatory action, β thromboglobulin, IL-1β, FV, FVIII, FXI, HMWK factors, C1-inhibitor, Tissue Factor Pathway Inhibitor (TFPI), protein S, proteins with anti-microbial action and lastly the network of growth factors which become strategic for healing and reconstruction of the cell matrix.

The bioavailability of all these molecules derived from the haematopoietic environment influences the cell proliferation which depends only partly on the number of platelets present in the concentrate.

The plasma can be enriched with several proteins selected from the group consisting of:

| |
|---|
| ACTC- Cardiac alpha-actin |
| ACTBL - Beta-actin |
| COF1- Complement 1 |
| ACTG - Gamma-actin |
| F13A - Factor XIII |
| G3P - Glycoprotein 3p |
| FCN3 - Ficolin-3 |
| HSP7C - Heat Shock Protein 7 |
| GP1BA - Glycoprotein 1b |
| CO6 - Complement |
| CO5 - Complement |
| SPTC2 - Serine-palmitoyl-CoA transferase 2 |
| ALDOA - Fructose-bisphosphate aldolase A |
| GELS - Gelsolin |
| ALBU - Albumin |
| CO8B - Complement |
| CO9 - Complement |
| PEDF - Pigment epithelium-derived factor |
| C1RL - Complement |
| RSU1- Ras 1 suppressor |
| FA12 - Factor XII |
| PGRP2 - N-acetylmuramoyl-L-alanine amidase |
| HV551 - Immunoglobulin |
| ALS - Insulin-like growth factor binder |
| CBPN - Carboxypeptidase N |
| CBG - Transcortin |
| GSTO1- Glutathione S-transferase Omega 1 |
| FIBB - Fibrinogen |
| LV321 - Immunoglobulin |
| LV861 - Immunoglobulin |
| CO7 - Complement 7 |
| HV315 - Immunoglobulin |
| VTNC - Vitronectin |
| AACT - Alpha-1-antichymotrypsin |
| C1R - Complement |
| IGL1 - Immunoglobulin |
| PROF1 - Profilin-1 |
| KV224 - Immunoglobulin |

According to a third aspect of the disclosure a cell culture medium is provided comprising a plasma enriched with platelets and plasma proteins as described above, in particular, obtained via the steps of:
- obtaining a platelet-rich plasma by means of centrifugation or extraction from anticoagulated whole blood or from apheresis platelet concentrate;
- filtering said platelet-rich plasma on hollow fibres of polysulfone or derivatives thereof having an outer surface provided with pores having mean diameter ranging from 5 µm to 15 µm and an inner surface provided with pores having mean diameter ranging from 5 nm to 80 nm to obtain a plasma enriched with platelets and plasma proteins.

### BRIEF DESCRIPTION OF THE DRAWINGS

The present invention will now be described in detail with reference to the figures of the attached drawings, which show purely illustrative and non-limiting embodiment examples, in which:
- Figure 1 schematically illustrates the procedure followed to obtain the plasma enriched with platelets and proteins according to the present invention;
- Figure 2 illustrates the results of the proliferation of fibroblasts grown in a medium supplemented with blood components of various types;
- Figure 3 illustrates the results of the proliferation of fibroblasts grown in a medium supplemented with the plasma enriched with platelets and proteins obtained with the method according to the present invention;
- Figure 4 illustrates the growth factor content of platelet-rich plasma (PRP) at different concentrations;
- Figure 5 illustrates the increase in plasma proteins in samples of the plasma enriched with platelets and proteins obtained with the method according to the present invention.

### BEST MODE FOR CARRYING OUT THE INVENTION

Further characteristics of the present invention will become clear from the following description of some merely illustrative non-limiting examples.

### Examples

### Preparation of the platelet-rich plasma (PRP)

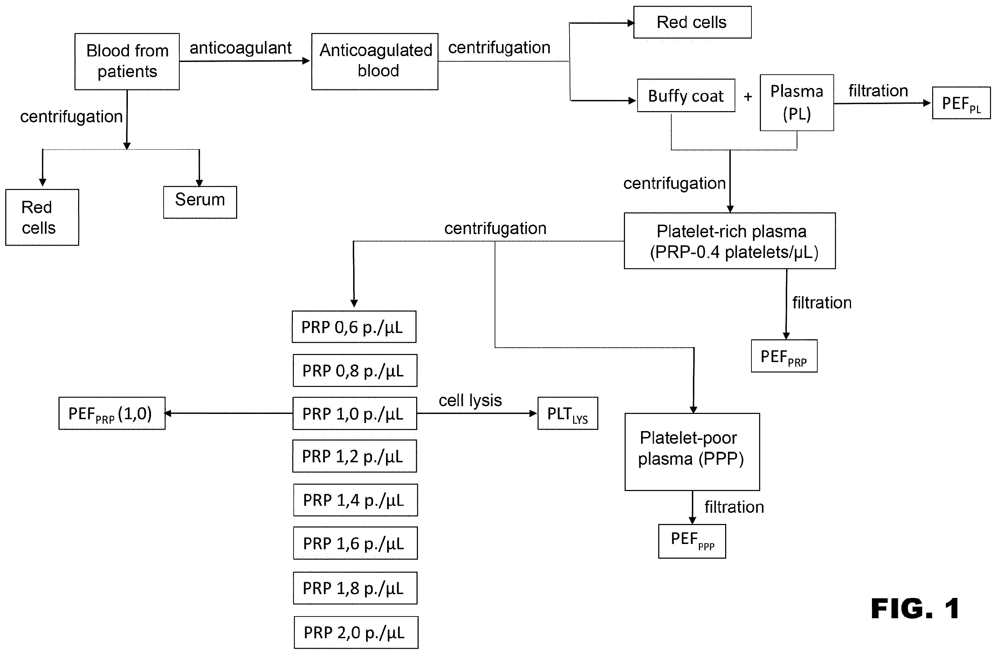

A diagram of the method used to produce the blood components tested in the following examples is shown in Figure 1.

The blood samples were collected from 10 volunteer donors and two pools were prepared: one of anticoagulated blood to produce, by means of centrifugation, a platelet-rich plasma (PRP) (CPunT system - Eltek Group SpA - Hone Italy) and one of blood without anticoagulant to obtain, by means of centrifugation, serum (VI 2PRP Biomed Device test tubes-Modena). The 2x concentrated PRP (PRP 0.4 x 10⁶ platelets_{/}µL) was obtained via low speed centrifugation (156 rcf x 10' Heraeus Megafuge 16 ThermoFisher Scientific - Germany), and processed and separated automatically. 0 mL of PRP were collected: 20 mL were centrifuged (1200 rcf x 10') to obtain the concentrations: 0.6, 0.8, 1.0, 1.2, 1.4, 1.6, 1.8, 2.0 x 10⁶ platelets_{/}µL via appropriate modulation of the supernatant plasma volume, the other 20 mL were filtered to obtain the PEF_{PRP}. The platelet-poor plasma (PPP) was collected after the second centrifugation of the PRP (PRP 0.4 see Fig. 1) and subsequently split into two portions, one of which was filtered to obtain the PEF_{PPP}. All the preparations were evaluated for corpuscle content by blood count (ABX Micro ES 60 - Horiba Medical Group) and for the number of platelets, a tolerance range of ±0.5% was accepted; all the preparations were portioned and stored at -40°C.

### Enrichment of the blood components by filtration: preparation of PEF_{PRP}, PEF_{PPP}, PEF_{PL}

The part of PRP 0.4 platelets/µL (20mL) and of the different blood components (PPP and PL) was filtered with the ProtSMART^{™} filter (Medica Group - SpA, Medolla - Italy). This is a medical device (CE) which through capillary fibres of Medisulfone^{®} (15 kDa mean cut-off of fibres; mean porosity 5 nm) allows a concentrate of protein-rich platelets (PEF_{PRP}) to be obtained, starting from a platelet-rich plasma. The filter is connected to the syringe that contains the product to be filtered (7 to 20 mL maximum) and due to the pressure exerted slowly and constantly on the syringe, the blood component is concentrated by dead-end filtration. The filtering elements are transferred in a sterile manner (Laminar Flow Cabinet Biohazad - Bluebam 4, Blueair - Italy) into syringes: the filtration/concentration of the blood component is carried out according to the manufacturer's instructions. The concentrated product is recovered by suction through the same filter inlet path (2-8 mL). After filtration the platelets are functioning (PFA-Siemens Germany), morphologically intact and non-aggregated (Leica DFC 295 microscope). The concentration is 4-5 times the basal value and the plasma is concentrated and enriched with proteins (at least twice the basal value). On a portion of each blood component before and after filtration, the total proteins were assayed (ADVIA 2400 Siemens - Germany) also on the water extracted.

The plasma (PL) was obtained from anticoagulated blood (VI 1PRP test tubes with ACD Biomed Device - Modena) separated by centrifugation (1200 rcf x 10') and split into two portions; one was filtered to obtain the PEF_{PL}.

The platelet-poor plasma (PPP) obtained as described above and collected after the second centrifugation of the PRP (PRP 0.4 see Fig. 1) was filtered with Medisulfone^{®} to obtain the PEF_{PPP}.

The PRP obtained as described above was filtered with Medisulfone^{®} to obtain the PEF_{PRP}.

### Serum

The test tubes without anticoagulant (test tubes VI 2PRP Biomed Device - Modena) were centrifuged (2340 rcf x 15') and the serum extracted from each test tube was collected in a uniform pool.

### Platelet lysate (PLT_{Lys})

A portion of PRP 1.0 was delicately washed three times with PBS at 3% EDTA, the platelets were then re-suspended in PBS (volume equal to initial volume) and subjected to thermal shock -80°C/+37°C. The platelet membranes crushed by freezing were removed by high speed centrifugation. PLT_{Lys} was then frozen in portions at -40°C until the time of use. This preparation was used as the reference standard to verify the effect of the molecules extracted from the platelets and therefore avoid interaction interference with those of the plasma.

### Quantification of the growth factors in PRP 1.0, PPP, PL, SERUM, PEF_{PRP}, PEF_{PPP} and PEF_{PL}

IGF-1, VEGF, EGF, PDGF-bb and TGF-β were quantified using the Quantikine^{®}ELISA kit (R&D Systems, Abingdon, UK). The assay was performed according to the manufacturer's instructions.

### Human dermal fibroblast cultures

Primary human fibroblasts were used (Normal Human Dermal Fibroblasts adult skin - Lonza Walkersville Inc. - USA) kept in complete culture medium (D-MEM with the addition of 2mM L-Glutamine, 100 IU/mL penicillin, 100 µg streptomycin and 0.5% amphotericin B - Invitrogen Corporation, USA) at 10% FBS. The growth and morphology of the cells were evaluated by observation under an optical microscope (Leica DMIL - Germany). The tests were performed in plates with 96 wells and at each session the results were related to a growth curve (defined standard). At day 0, 5000 cells per well were seeded (day 0) in complete culture medium, after 24 h (day 1), once adhesion had taken place, the medium was replaced with complete medium without FBS, in order to induce synchronization of the seeded cells and avoid further interferences from plasma of animal origin.

### Stimulation of cell proliferation by means of the blood component preparations

At 4 days from seeding, the medium was replaced with medium without FBS added to 10% of the blood component preparations. The cultures were then set up for verifying the effect of: PL, PEF_{PL}, PPP, PEF_{PPP}, PRP 1.0, PEF_{PRP} (1.0), SERUM and PRP at scaled and increasing platelet concentration 0.4, 0.6, 0.8, 1.0, 1.2, 1.4, 1.6, 1.8, 2.0. The cultures were carried out at 37°C and 5% CO₂, in some experiments (n=8); in parallel, cultures were set up in the presence of expanded cells 10% FBS as standard additive.

### Short-term cell proliferation assay

For determination of the cell proliferation rate, the short term of 7 and 11 days from seeding was chosen, after stimulation with blood component preparations.

After prolonged washing with PBS, the cells were incubated at different times with a diluted solution of reagent solution (CellTiter 96 Aqueous One Solution cell proliferation assay Promega - USA) for 3 hours at 37°C in a humidifier, in 5% CO₂ atmosphere. The absorbance was recorded at 490 nm using a plate reader (Anthos 2010 GSG Robotics - Italy).

### Statistics

The different results between the cell culture conditions were established by means of the ANOVA method with the software GraphPad Prism 5 (GraphPad Software, Inc, San Diego, CA). The analyses were carried out using the ANOVA one-way and Dunnet post-test.

In the figures, the data are expressed as means and standard deviations.

### Results

The growth tests were carried out with medium at 10% FBS or 10% of the different blood component fractions, based on studies with growth curves obtained from stimulations at different percentages and published in the literature that defines this supplementation as effective (SH Zaky, A Ottonello, P Strada, R Cancedda: Platelet lysate favours in vitro expansion of human bone marrow stromal cells for bone and cartilage engineering- Journal of tissue engineering and regenerative12/2008).

9 portions of PRP were prepared with a number of platelets from 0.4 x10⁶ (twice the mean basal value of PLT in the peripheral blood) up to 2.0 x10⁶/µL as supplement to the complete medium replacing the FBS, to verify whether the platelet concentration variation in the PRP influences cell growth. All the results were reported in a graph and all the concentrations were compared as a percentage variation relative to the PLT_{Lys}.

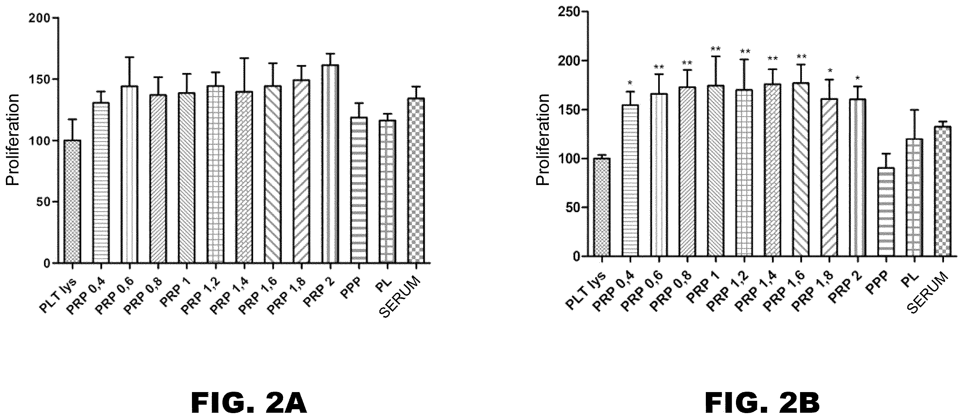

The cells were seeded (to guarantee correct adhesion and vitality) and then stimulated after 72 hours from seeding with the different blood components. At 7 days from the seeding (Figure 2a) the growth evaluations were carried out and no statistically significant differences in proliferation were highlighted between the reference condition, PLTLys, and the different treatments.

Repeating the treatment as previously, but maintaining the supplementation up to 11 days from seeding (Figure 2b), it is observed that the proliferation induced by all the different PRP concentrations is significantly higher than the reference proliferation, whereas this is not so for the values reached after treatment with PPP, PL and serum.

The results have shown that the number of platelets influences the cell growth, although not in a linear manner and without a direct proportionality. In particular, calculating the number of cells, it is observed that *in vitro,* also at low concentrations (0.4 x10⁶/µL), the platelet concentrate has an inductive effect on growth, but the best effect is achieved when the concentration is between 4 and 8 times (0.8 x10⁶ and 1.6 x 10⁶/µL) the basal value.

Figure 2 shows the proliferation data resulting from the use of a medium with the addition of PRP with platelet concentration 1x10⁶/µL compared with all the fractions obtained from the various PRP work and preparation phases. A complete medium with FBS was used as a control (CTRL+ FBS). The filtration was added as an innovative method to the preparation of these blood components and the concentrates obtained were not only enriched with platelets but also with total proteins (Table 1).

**Table 1**

| Proteins | PRP 0.4 | PEF_{PRP} | PL | PEF_{PL} | PPP | PEF_{PPP} | Serum | Water |
|---|---|---|---|---|---|---|---|---|
| g/dL | 6 ± 0.2 | 17,5 ± 1.4 | 6.1± 0.1 | 15,7 ± 1.2 | 6.1 ± 0.2 | 10.7± 0.9 | 6.9 ± 0.1 | 0.0 |

The evaluation was carried out at 7 days and 11 days from seeding, since it had already been observed that the highest growth increase occurs from day 7.

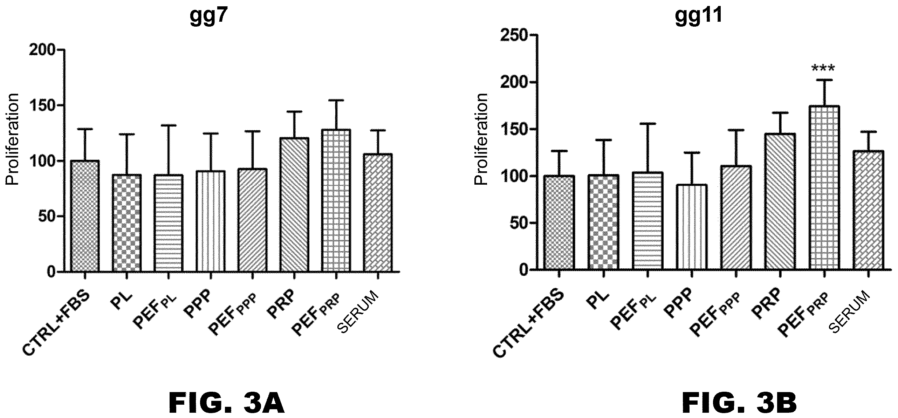

At day 7, none of the treatments caused a significant increase in proliferation (Figure 3) whereas after 11 days of treatment, only the PEF_{PRP} demonstrated its ability to induce a significant increase in proliferation with an increase of approximately 50%, compared to the control (CTRL+FBS).

The results confirm that the PRP increases cell proliferation, obtaining a double growth compared to the control; the serum maintains a positive effect just above the control. The most intense proliferation is the one sustained by the PEF_{PRP}.

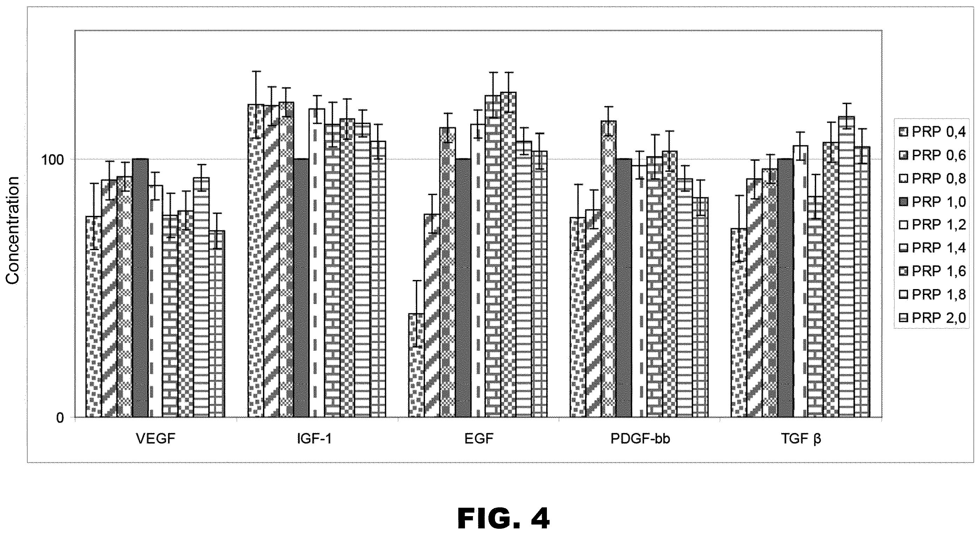

A low correlation was found between the dosable content of the GFs and the platelet concentration of the PRP. The IGF-1, which is a plasma protein, is relatively uniform in all the products. For the other factors, a certain scalarity is noted from PRP 0.4 to PRP 1.0, whereas at concentrations higher than PRP 1.4, almost a paradox effect is observed which can be attributed to a polymerization of the GFs beyond a certain concentration (Figure 4).

Table 2 shows the concentrations of the GFs obtained in the different formulations.

**Table 2**

| pg/mL | IGF-1 | VEGF | EGF | PDGF-bb | TGF β |
|---|---|---|---|---|---|
| PRP 1.0 | 165 ± 8 | 585 ± 75 | 2670 ± 186 | 34526 ± 283 | 2649 ± 185 |
| PPP | 233 ± 50 | 214 ± 83 | 104 ± 17 | 4558 ± 2204 | 786 ± 51 |
| PL | 274 ± 22 | 246 ± 4 | 109 ± 54 | 2597 ± 634 | 679 ± 21 |
| SERUM | 438 ± 27 | 405 ± 21 | 1444 ± 369 | 14493 ± 1443 | 933 ± 95 |
| PEF_{PRP} | 369 ± 141 | 786 ± 175 | 3965 ± 496 | 115675 ± 6088 | 1448 ± 112 |
| PEF_{PPP} | 353 ± 92 | 173 ± 127 | 247 ± 148 | 5408 ± 826 | 705 ± 71 |
| PEF_{PL} | 404 ± 54 | 241 ± 49 | 147 ± 102 | 4221 ± 413 | 679 ± 11 |

The filtration allowed a greater quantity of GFs to be recovered in all the preparations, in particular the PEF_{PRP} increases the concentration by at least 1.5/2 times compared to the PRP.

The IGF-1, as a plasma protein, is expressed to a greater extent in the PPP, PL and respective filtrates, whereas in the PRP it is lower but becomes concentrated with the filtration; in the serum the value equals PEF_{PRP}.

VEGF, EGF, PDGF-BB and TGF-β are platelet release values and they all reflect the same trend: quantitatively lower, as predicted, in PPP, PL and respective filtrates, whereas the PDGF-BB and EGF in particular are well expressed in PRP and PEF_{PRP}. In the serum the quantities are high, being by definition obtained from retraction of the coagulate, but lower with respect to the PEF_{PRP}.

### DISCUSSION

The topical administration of platelet-rich plasma is a procedure frequently used for the treatment of numerous clinical conditions that require tissue repair improvement. This requires synergy and redundancy between the mechanisms involved to generate appropriate and stable results. The majority of the cell activities are responses to numerous signals and each signal causes more than one activity. The redundancy of multiple signals is a general role in the development and repair of a tissue.

The object of the application of the blood components for non-transfusional use in Regenerative Medicine is to provide biomolecules and proteins physiologically already present in the tissues, but which due to intrinsic or extrinsic factors are insufficient since they are affected by any inhibition at local level, or are completely absent, slowing down the healing process. The platelets are natural reservoirs of different factors with anabolic and catabolic synergic functions. Therefore, their administration in a concentration higher than the basal level provides the right factors in the right proportion to accelerate the cell response and obtain regeneration of the tissue.

The ratio between platelet concentration and release of GFs has already been evaluated in many studies, in particular in works that compare different preparation methods. The same inventors published in 2009 a comparison between 4 types of preparations. The sole quantitative evaluation of the GFs released/present in the different formulations of platelet concentrates is only a preliminary step for predicting their clinical effectiveness *in vivo.* This study has evaluated the effect on cell proliferation induced by blood components enriched with proteins compared to that induced by some products already used in numerous clinical conditions.

Filtration of the PRP was introduced as an innovative method to obtain, through a process which is non-selective at cell level but deprives the plasma of its aqueous component, a greater concentration of the platelets and plasma proteins. Filtration is an alternative to centrifugation which is normally applied to increase the platelet concentration of the PRP. Compared to centrifugation, it is less aggressive on the platelets and preserves their structure and function more effectively. Furthermore, in addition to allowing a greater numerical and functional recovery of cells, it enriches their protein content.

Evaluation of the effect on cell growth of platelet concentrations with different PRP has confirmed data already published previously by several authors.

The platelets release into the plasma quantities of bioactive molecules roughly proportional to the platelet concentration in the preparation. The effect on proliferation of the target cells induced by the variable quantities of factors released by the platelets is illustrated in Fig. 4. It can be affirmed for certain that the release of factors by the platelets is an essential and basic component in relation to the stimulation of cell proliferation; the importance of the biological effect by the PRP preparations is evident when comparing the induced proliferation relative to that of the plasma (which contains plasma proteins but is virtually without activatable platelets), with the platelet lysate (which contains platelet derivation factors but is without plasma molecules) and the serum (which contains residual coagulation factors and cell component release molecules "trapped" in the coagulate). In fact, the addition to the medium of plasma, serum or lysate stimulates cell proliferation in a way not dissimilar to that obtained in the control (complete medium with 10% FBS) while all the PRP, whatever their platelet concentration, produce a greater stimulus. Due to the fact that the proliferation of the target cells is sensitive to the twin trigger mechanisms (plasma proteins and platelet derivation factors), it is not directly correlated with the sole platelet concentration. This is a new element: in fact, it has always been thought that, within certain limits, the cell growth induced by the platelets is always correlated with their concentration. However, the inventors have highlighted that said linear relation occurs only up to the maximum concentration of platelets equal to 1.4 x10⁶ µL (PRP 1.4).

At higher concentrations, the cell proliferation decreases, showing a Gaussian relationship between platelet concentration and proliferative response. Although tissue regeneration studies performed *in vivo* attribute to 1x10⁶µL (PRP 1.0) maximum clinical effectiveness of the platelet concentrates, the data relative to the present invention show that at the same platelet concentration in the different preparations, the cells in culture respond differently, and in particular, where there is a greater concentration of proteins (PEF_{PRP}) the response is greater and statistically significant.

The plasma proteins in the preparations without the platelet component (PL and PPP), in the cell model used here, induce a proliferation equal to the control both at 7 days and at 11 days from seeding, also after filtration (PEF_{PL} and PEF_{PPP}). Although the concentration of total proteins is greater, the cell growth does not increase as presumed; the effect of the serum, although slightly greater, is analogous.

From these considerations it can be affirmed that the significant increase induced at day 11 by the PEF_{PRP} is due to the cooperation of the action of the plasma proteins and platelet proteins.

The platelets are anucleate cells but specialized in secreting the content of their intracellular granules (which also contain auto-synthesized proteins) in response to stimuli.

The granules contain, together with vesicles with membrane, prothrombin release proteins, mitogenic proteins, immunomodulatory proteins and adhesive proteins, growth factors, signal molecules, cytokines, integrins and coagulation proteins. Among the proteins in the PRP there are complement factors (C1RL, CO6, CFAH, CO3, CFAB, C1QA, CO4B), immunoglobulins (LV101, LV107) and serine proteases (THBG, CBG). The latter are the main components of the coagulation and complement cascade present in the plasma.

Many of these proteins, which are found in the granules α, are present in the plasma but some coagulation factors that derive from platelets are structurally different from the corresponding factors present in the plasma.

This suggests that some plasma proteins and the factors released by the platelets cooperate synergically with induction of the proliferative response in the target cells and that said synergic action could depend on the concurrence of various activation paths of the cytoplasm mechanisms; these converge on the signal transduction paths triggering the cell proliferation network (gene activation, mitosis, protein synthesis) and therefore give rise to tissue regeneration.

### CONCLUSIONS

The results of the study show that the relation between the proliferation of primary fibroblasts in vitro and the concentration of platelets in the conditioning medium is not strictly linear, in particular it has been seen that the difference in a range between 0.6 and 1.6 x 10⁶ platelets/µL does not induce a significantly different cell growth result. The PRP produced by filtration (PEF_{PRP}) induces a greater cell growth than any other product tested. The probable lower platelet stress which is induced during filtration results in a considerable increase in the available fraction of growth factors.

### Comparison and identification of the proteins detected with proteomic analysis in the PEF_{PRP} and PRP

A proteomic analysis was performed on the PRP and on the PEF_{PRP} to identify the main plasma proteins.

The products analysed are:
A) PRP (platelet concentration: 1x10⁶ per µL)
B) PEF_{PRP} (filtered platelet concentrate obtained from a portion of PRP 1x10⁶ platelets/µL)

The proteomic analysis performed on A and B aimed at identifying the most expressed proteins involved in the tissue regeneration mechanisms. Approximately 80 proteins per product were identified and then compared.

The comparison, aimed at evaluating how the filtration on Medisulfone^{®} modulates the concentration of these proteins, was performed via a dedicated specific software.

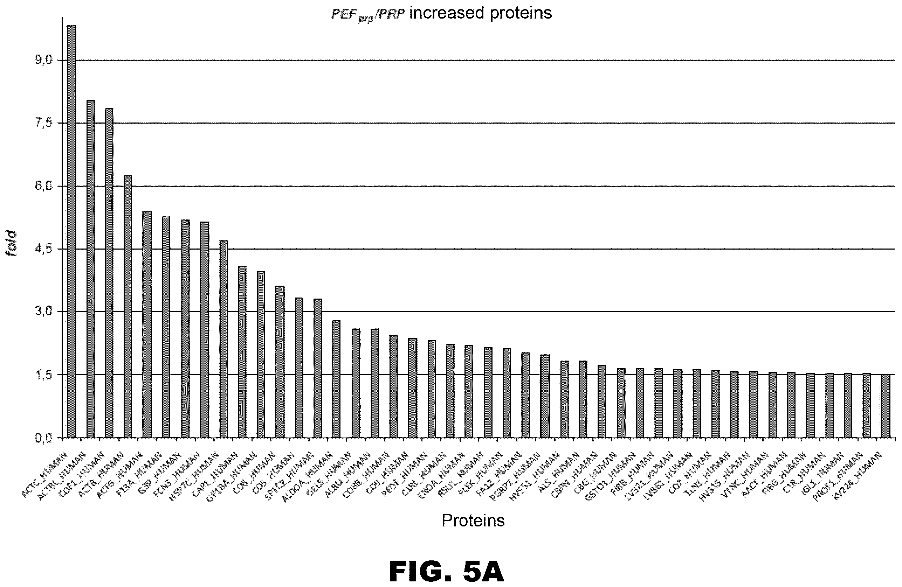

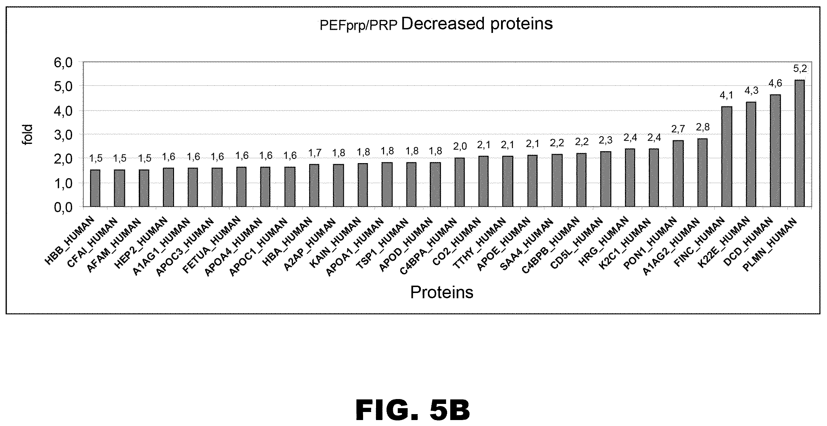

The results, given in Figure 5a and 5b, show an increase of 44 proteins in the filtrate compared to the corresponding PRP (Figure 4a, fold change = how many "times" - statistically significant - the protein has a greater concentration in the PEF_{PRP} than in the PRP) and a reduction in the presence of the other 33 proteins (Figure 4b).

Table 3 shows the up- and down-regulated proteins grouped with reference to the main effects in the wound healing process.

**Table 3**

| PROCESS | UP-regulate | DOWN-regulate |
|---|---|---|
| Inflammatory | CBG, GSTO1 | CD5L |
| Anti-Inflammatory | CO5, AACT, GSTO1, CBPN | HRG, A1AG1, A1AG2, KAIN |
| Wound healing (proliferation, angiogenesis, interaction with GF, apoptosis) | ACTB, ACTG, FCN3, TNL1, HSP7C, ACTC, SPTC2, CBPN, GELS, ENOA, VTNC, ACTC, COF1, CAP1, ALDOA PROF1, PEDF, FCN3, AACT | FETUA, APOA1, FINC, K22E, DCD, TSP1, KAIN |
| Stabilization of the coagulate | FA12, FIBG, F13, FIBB | APOC1, APOC, APOA4, APOD |
| Antimicrobial | PRGP2 | DCD |

Proteins of the plasma component were then identified (fractions of the Complement and Immunoglobulins) and of the platelet membranes as platelet antigens (membrane Glycoproteins). The results are given in Table 4.

**Table 4**

| | | |
|---|---|---|
| Complement | CO8B, CO7, CO6, | CFA1, C4BPA, C4BPB |
| Immunoglobulins | HV551, LV861, KV224, HV315 | |
| Platelet antigens | GP1, GP3 | |

## Claims

1. A method for preparing plasma enriched with platelets and plasma proteins having a platelet content of at least 600000 platelets/µL and a plasma protein content of at least 10 g/dL, **characterised in that** it comprises the steps of:
- obtaining a plasma enriched with platelets by means of centrifugation or extraction from anticoagulated whole blood or from platelet concentrate from apheresis;
- filtering said plasma enriched with platelets through hollow fibres of polysulfone or derivatives thereof having an external surface provided with pores of average diameter comprised between 5 µm and 15 µm, and an internal surface provided with pores of average diameter comprised between 5 nm and 80 nm for obtaining a plasma enriched with platelets and plasma proteins.

## Patentansprüche

1. Verfahren zur Herstellung von mit Blutplättchen und Plasmaproteinen angereichertem Plasma mit einem Blutplättchengehalt von mindestens 600000 Blutplättchen/µL und einem Plasmaproteingehalt von mindestens 10 g/dL, **dadurch gekennzeichnet, dass** es die folgenden Schritte umfasst:
- Gewinnen eines mit Blutplättchen angereicherten Plasmas durch Zentrifugation oder Extraktion aus antikoaguliertem Vollblut oder aus Blutplättchenkonzentrat aus der Apherese;
- Filtrieren des mit Blutplättchen angereicherten Plasmas durch Hohlfasern aus Polysulfon oder Derivaten davon mit einer äußeren Oberfläche, die mit Poren mit einem durchschnittlichen Durchmesser zwischen 5 µm und 15 µm versehen ist, und einer inneren Oberfläche, die mit Poren mit einem durchschnittlichen Durchmesser zwischen 5 nm und 80 nm versehen ist, um ein mit Blutplättchen und Plasmaproteinen angereichertes Plasma zu erhalten.

## Revendications

1. Procédé de préparation de plasma enrichi en plaquettes et en protéines plasmatiques ayant une teneur en plaquettes d'au moins 600 000 plaquettes/µL et une teneur en protéines plasmatiques d'au moins 10 g/dL, **caractérisé en ce qu'**il comprend les étapes suivantes :
- l'obtention d'un plasma enrichi en plaquettes par centrifugation ou extraction à partir de sang total anticoagulé ou de concentré de plaquettes d'aphérèse ;
- la filtration dudit plasma enrichi en plaquettes à travers des fibres creuses en polysulfone ou ses dérivés ayant une surface externe pourvue de pores de diamètre moyen compris entre 5 µm et 15 µm, et une surface interne pourvue de pores de diamètre moyen compris entre 5 nm et 80 nm pour obtenir un plasma enrichi en plaquettes et en protéines plasmatiques.
